# EUROPEAN PATENT APPLICATION

(11) **EP 2 514 722 A1**
(43) Date of publication of application: **24.10.2012**
(21) Application number: 11163045.5
(22) Date of filing: 19.04.2011
(51) Int. Cl.: C02F 3/34

(54) **Composition and process for the treatment of waste produced by oil mills**

(71) Applicant: Spechim S.A., 1410 Waterloo (BE)
(72) Inventor: Decoster, Rudy, 1410, WATERLOO (BE)
(74) Representative: Pecher, Nicolas

(57) **Abstract**

Process and composition for treating oil mill waste(water), the composition comprising at least one polyphenol-resistant bacterium, pref. a Bacillus, and an enzyme, pref. selected from protease, amylase, alcalase and neutrase. A fungus, pref. Basidiomycetes, and a base for pH regulation may be added. The target contaminant may be alpechin.
An installation comprising extracting and separating means as well as a lagoon is also claimed.

## Description

### TECHNICAL FIELD

The present invention relates generally to the field of waste produced by oil mills. In particular, the present invention relates to the treatment of alpechin, the waste products resulting from the process of extracting oil at the oil mills.

### BACKGROUND OF THE INVENTION

Vegetal oil, such as olive oil, is produced by grinding fruits or plants and extracting the oil by mechanical or chemical means. For example, olive oil extraction produces large amounts of residues. Liquid wastes produced by mills are known as alpechin. Alpechin-polluted waste waters are considered as a severe environmental problem because of their high organic content. Indeed, alpechin is constituted of water, organic and mineral compounds, in particular phenolic compounds such as polyphenols that are antimicrobial and phytotoxic.

For example, about 200 liters of alpechin are produced from one ton of olive. Olive oil waste effluents are major pollutants and constitute a great problem in olive tree cultivation areas. In most countries, it is not allowed to dispose of the wastes directly into the waterways, and they have to be stored in closed or open ponds. Closed ponds are sources of contamination for surface and ground waters while in open ponds many volatile malodorous compounds and large areas of arable land are necessary.

Various strategies can be applied when dealing with alpechin-polluted waste waters: use, eliminate or purify wastes. Use of alpechin as a fertilizer or bioenergy production was previously suggested (Ramos-Cormenzana et al., International Biodeterioration & Biodegradation, 1995, 249-268).

Purification of the alpechin-polluted waste waters can also be conducted by filtration and/or by enzymatic process as disclosed in ES 2 110 912.

EP 0 718 397 discloses a process for the purification of liquid and solid waste products produced by oil mills. The process comprises a series of physical processes to provide a solid or liquid suitable as fertilizers and useful in a composting plant or to make fodders.

ES 2 239 914 discloses a plant and a process for the treatment of the alpechin-polluted waste waters. The process comprises the steps of physical separation of sludge and fat, oxygenation of alpechin, chemical treatment to lower the phenol content and filtration of the residue. The chemical treatment consists of a saponification step and addition of a chloride component.

WO 92/11206 discloses a process for purifying agricultural waste liquid from the processing of fruit. The process comprises filtration steps and contacting water-soluble waste with a flocculating agent such as chitosan.

The processes disclosed in the art, however, are not able to sufficiently purify the waste waters in such a way that the water could be reused and rejected in waterways. Physical and chemical processes to purify and detoxify alpechin have met limited success and were not shown to be cost-effective.

The present invention aims at providing a composition and a process that overcomes the above-discussed drawbacks of the prior art.

### SUMMARY OF THE INVENTION

In particular, it is an object of the present invention to provide a composition and a process able to treat waste products produced by oil mills, such as alpechin-polluted waste waters. It is another object to provide a process treating alpechin-polluted waste waters to produce water having a chemical oxygen demand (COD) lower than 125 mg/I.

In a first aspect of the present invention, a kit of parts for the treatment of waste products produced by oil mills is provided. Said kit of parts comprises at least one polyphenol-resistant bacterium and enzymes. Preferably, said at least one bacterium may comprise a bacterium from *Bacilli* class. Said kit of parts may further comprise one or more fungi, preferably from *Basidiomycetes* class. Said waste products produced by oil mills may be alpechin-polluted waste waters.

In a second aspect of the present invention, a process for the treatment of waste product produced by oil mills is provided. Said process comprises the steps of:
(a) contacting the waste product with at least one polyphenol-resistant bacterium to form a waste solution,
(b) adding enzymes and, optionally, a pH regulator to maintain the pH of the solution between 6 and 9,
(c) adding one or more fungi from *Basidiomycetes* class, and let rest for a time sufficient to provide a treated-waste product having a chemical oxygen demand lower than 125 mg/l.
Preferably, the treated-waste product obtained at the end of the process may have a chemical oxygen demand lower than 110 mg/l. Said waste products produced by oil mills may be alpechin-pollutant waste water.

In another aspect, the present invention provides a system for the treatment of waste products produced by oil mills, comprising extracting means, separating means, a treatment basin and a lagoon.

In a further aspect, the present invention also provides a composition comprising waste products produced by oil mills, enzymes and at least one polyphenol-resistant bacterium. Preferably, said at least one bacterium may be from *Bacilli class.*

The present kit of parts and process allow the treatment and the purification of waste product produced by oil mills. Such a treatment is performed without physical purification such as filtration, or chemical purification such as redox reactions. The present invention allows the purification of waste waters to acceptable quality at the end of the process, even reaching drinking water standards. The treated-waste product was constituted of 95% of water and 5% of inert residue. Therefore, the present invention is a simple and cost-effective process for the purification of alpechin-polluted waste products, such as alpechin-polluted waste water. The terms "alpechin-polluted waste water" and "alpechin-polluted waste products" can also be named simply "alpechin" in the following.

### BRIEF DESCRIPTION OF THE DRAWING

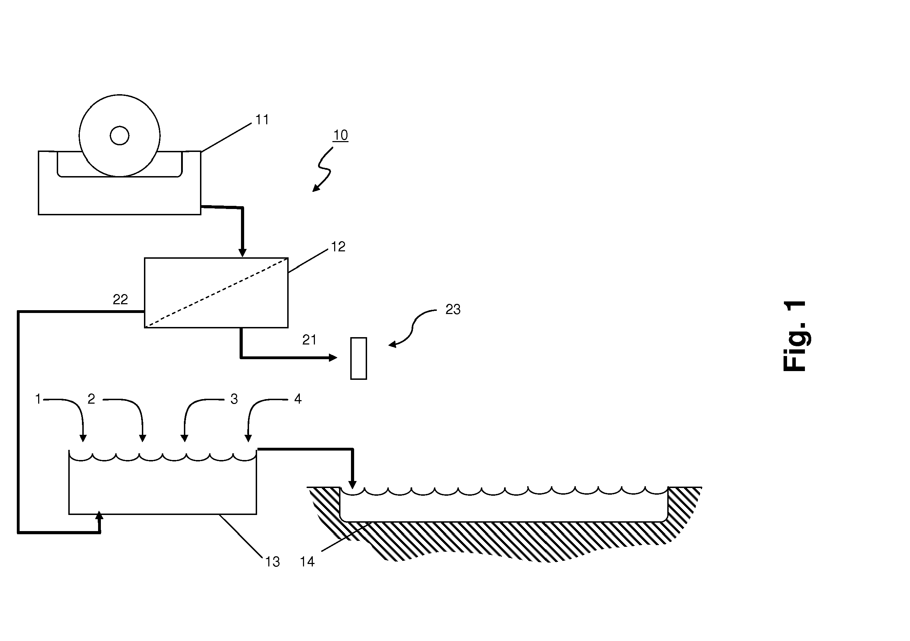

Fig. 1 represents a schematic view of the system and of the process of the present invention for the treatment of alpechin.

Fig. 2 represents a graph representing the chemical oxygen demand in function of time according to the present invention.

### DETAILED DESCRIPTION OF THE INVENTION

In a first aspect, the present invention provides a kit of parts comprising at least one polyphenol-resistant bacterium (1), and enzymes (2). The kit of parts may comprise one or more than one polyphenol-resistant bacterium (1). In particular, it may comprise 1, 2, 3, 4, 5, 6, 7, 8, 9, 10, 15, 20 polyphenol-resistant bacteria or a number between any of two of the aforementioned values. The term "polyphenol-resistant" as used herein refers to a bacterium for which the polyphenol compounds have limited bacterium growth inhibition effect. The polyphenol-resistant character of the bacterium may be determined by a method known in the art such as disc diffusion method (Bauer et al., 1966, Am. J. Clin. Pathol., 45, 493-496).

Said at least one polyphenol-resistant bacterium (1) may comprise a bacterium from *Bacilli* class, preferably from *Bacillales* order. More preferably, said at least one polyphenol-resistant bacterium (1) may comprise a bacterium selected from the *Bacillaceae* family. Most preferably, said at least one bacterium (1) may comprise a bacterium selected from the *Bacillus* genus.

Non-limitative examples of bacteria from *Bacillus* genus that may be suitable for the present invention are *Bacillus alcalophilus, Bacillus alvei, Bacillus amyloliquefaciens, Bacillus aneurinolyticus, Bacillus anthracis, Bacillus aquaemaris, Bacillus brevis, Bacillus caldolyticus, Bacillus centrosporus, Bacillus cereus, Bacillus circulans, Bacillus coagulans, Bacillus firmus, Bacillus flavothermus, Bacillus fusiformis, Bacillus globigii, Bacillus infernus, Bacillus larvae, Bacillus laterosporus, Bacillus lentus, Bacillus licheniformis, Bacillus megaterium, Bacillus mesentericus, Bacillus mucilaginosus, Bacillus mycoides, Bacillus natto, Bacillus pantothenticus, Bacillus polymyxa, Bacillus pseudoanthracis, Bacillus pumilus, Bacillus schlegelii, Bacillus sphaericus, Bacillus sporothermodurans, Bacillus stearothermophilus, Bacillus subtilis, Bacillus thermoglucosidasius, Bacillus thuringiensis, Bacillus vulgatis, Bacillus weihenstephanensis.*

In a preferred embodiment, said kit of parts may comprise a cocktail of at least four, preferably at least five, more preferably at least six bacteria wherein at least one bacterium is selected from the *Bacillus* genus.

In a preferred embodiment, said kit of parts may further comprise one or more gram-positive bacterium. Said one or more gram-positive bacterium may be selected from *Actinobacteria* phylum, preferably from *Actinobacteria* class. Non limitative examples of sub-class from *Actinobacteria* class are *Acidimicrobidae, Actinobacteridae, Coriobacteridae, Rubrobacteridae,* or *Sphaerobacteridae.* In particular, said one or more gram-positive bacterium may be selected from *Actinobacteridae* sub-class, preferably from *Actinomycetales* order, more preferably from *Nocardiaceae* family, most preferably from *Rhodococcus* genus.

Said kit of parts may further comprise one or more gram-negative bacterium. Said one or more gram-negative bacterium may be selected from *Proteobacteria* phylum, preferably from *Gammaproteobacteria* class. Non limitative examples of orders from *Gammaproteobacteria* class are *Enterobacteriales, Vibrionales, Pseudomonadales, Legionellales, Pasteurellales, Xanthomonadales, Thiotrichales, Oceanospirillales, Methylococcales, Chromatiales, Acidithiobacillales, Aeromonadales, Alteromonadales, Cardiobacteriales.* In particular, said one or more gram-negative bacterium may be selected from *Pseudomonadales* order, preferably from *Moraxellaceae* family, more preferably from *Actinobacter* genus.

In particular, said at least one polyphenol-resistant bacterium (1) may be a cocktail of at least five bacteria from *Bacillus* genus. Said cocktail may comprise two strains from *Bacillus subtilis,* one strain from *Bacillus lichenoformis* and one strain from Bacillus *sporothermodurans.* More particular, said at least one bacterium (1) may be a cocktail comprising *Rhodococcus erythropolis, Acinetobacter johnsonii,* and five bacteria from *Bacilli* genus. In particular, said kit of parts may comprise a cocktail of at least four bacteria selected among *Bacillus subtilis ATB-BS-10, Bacillus subtilis ATB-BS-13, Bacillus lichenoformis ATB-BS-14, Bacillus sp. ATB-BS-12, Bacillus sp. ATB-BS-15, Rhodococcus erythropolis,* and *Acinetobacter johnsonii,* and preferably comprising at least one *Bacillus* genus bacterium.

In a preferred embodiment, said kit of parts further comprises one or more fungi (3), preferably from *Basidiomycetes* class. For example, the kit of parts may comprise 1, 2, 3, 4, 5, or 10 fungi from *Basidiomycetes* class, or a number between any two of the aforementioned values. Preferably, said one or more fungi (3) may be from *Phanerochaetacae* family, more preferably from *Phanerochaete* genus. Non limitative examples of fungi (3) from Phanerochaete genus suitable for the present invention are *Phanerochaete allantospora, Phanerochaete arizonica, Phanerochaete avellanea, Phanerochaete burtii, Phanerochaete carnosa, Phanerochaete chrysorhizon, Phanerochaete chrysosporium, Phanerochaete radicata, Phanerochaete salmonicolor, Phanerochaete tuberculata, Phanerochaete velutina.*

In particular, said kit of parts may comprise one fungus which may be *Phanerochaete Chrysosporium.*

A kit of parts according to the present invention comprises enzymes (2) selected from the group consisting of protease, alcalase, amylase, and neutrase or mixtures thereof. Said kit may comprise 1, 2, 3, 4, 5, 10, or 15 different enzymes (2) or a number between any two of the aforementioned values. Preferably, said kit may comprise between 1 and 5 different enzymes (2). In a preferred embodiment, said enzymes (2) may be selected from the group consisting of amylase and neutrases or mixture thereof.

Non limitative examples of amylases belonging to EC 3.2.1 class and suitable for the present invention are alpha-amylase (EC 3.2.1.1), beta-amylase (EC 3.2.1.2), and gamma-amylase (EC 3.2.1.3). Preferably, said amylase may be an alpha-amylase. Amylases are commercially available as powder, granula, suspension or liquid solutions. Amylases are suitable to catalyse the breakdown of starch into sugar. Amylase may be produced from *Bacillus amyloliquefaciens.*

Neutrase is a bacterial neutral protease. For example, neutrase may be produced by a selected strain of *Bacillus amyloliquefaciens* or *subtilis.* Neutrase is a metalloendopeptidases belonging to class EC 3.4.24.

A kit of parts of the present invention may further comprise a pH regulator (4). The pH regulator (4) allows maintaining a medium suitable for the enzyme activity. For example, the pH regulator (4) may allow maintaining a pH between 6 and 9. Said pH regulator (4) may be a base such as hydroxide of alkali metals and alkaline earth metals or carbonate salts. Non limitative examples of suitable base are sodium hydroxide, potassium hydroxide, calcium hydroxide, barium hydroxide, lithium hydroxide, sodium carbonate, potassium carbonate, calcium carbonate, magnesium carbonate, lithium carbonate.

Said kit of parts of the present invention may further comprise nutrients for said at least one polyphenol-resistant bacterium (1). Said nutrients may allow producing a medium favourable to bacterium growth. Said nutrients are nutrients known in the art to favour growth of bacteria from *Bacilli* class. For example, nutrients may comprise calcium carbonate, sodium chloride, sorbitol, or saccharose or mixtures thereof.

A kit of parts of the present invention may further comprise urea. Urea may be useful to supplement the growth of said one or more fungi (3). Other nutrients may be used.

In another aspect of the invention, a composition is provided. Said composition comprises waste products produced by oil mills, enzymes (2) and at least one polyphenol-resistant bacterium (1). Preferably, said at least one polyphenol-resistant bacterium is from *Bacilli* class. In a preferred embodiment, said composition further comprises one or more fungi (3), preferably from *Basidiomycetes* class. Said composition may optionally comprise a pH regulator (4). Preferably, said waste products produced by oil mills may be alpechin-polluted waste waters. Said enzymes (2), at least one polyphenol-resistant bacterium (1) and said one or more fungi (3) are defined above with respect to the kit of parts. Said alpechin-polluted waste waters produced by vegetal oil mills may be issued from the production of cooking oil or vegetal fuel (23). Oil is extracted from plants or fruits. Alpechin may be issued from the production of various cooking oil such as olive oil, palm oil, soybean oil, canola oil, pumpkin seed oil, corn oil, sunflower oil, safflower oil, peanut oil, grape seed oil, or sesame oil. Preferably, alpechin may be a residue issued from the production of olive oil.

According to a third aspect of the present invention a process for the treatment of waste products produced by oil mills is provided. Said process comprises the steps of:
a) contacting the waste product with at least one polyphenol-resistant bacterium (1) to form a waste solution,
b) adding enzymes (2) and, optionally, a pH regulator (4) to maintain the pH of the solution between 6 and 9,
c) adding one or more fungi (3) from *Basidiomycetes* class, and let rest for a time sufficient to provide a treated-waste product having a chemical oxygen demand lower than 125 mg/l, preferably lower than 110mg/l.

In a preferred embodiment, the process may comprise the preliminary step of preparing a medium suitable for the growth of said at least one polyphenol-resistant bacterium (1).

In a preferred embodiment, the amount of said one or more fungi (3) to be added is at least 20g per cubic meter of waste products, such as alpechin.

In a preferred embodiment, the amount of enzymes (2) to be added is at least 50ml per cubic meter of waste product, preferably 100ml per cubic meter. Enzymes (2) may be added periodically. Preferably, enzymes (2) may be added once per day.

In a preferred embodiment, the amount of said at least one polyphenol-resistant bacterium (1) is at least 1kg per cubic meter of waste product, preferably 3kg per cubic meter of waste product. Preferably, said at least one bacterium (1) may be from *Bacilli* class as defined above.

In a preferred embodiment, enzymes (2), bacterium (1) and optionally pH regulator (4) as defined above are added before said one or more fungi (3). If the fungi are added at the beginning of the process, said fungi may be less efficient due to the high content of polluants in the waste solution. If the fungi are added too late, said fungi may not have sufficient time to degrade pollutant of the waste solution. Hence, said one or more fungi (3) may be added from 30 to 70 days after the beginning of the step a) of the present process, preferably from 40 to 60 days. More preferably, the one or more fungi (3) may be added c.a. 50 days after the beginning of the step a) of the present process.

The pH of the waste solution may be corrected continuously. The process of the present invention may further comprise the step of oxygenating the waste solution for at least 5 hours per day, preferably, 8 hours per day.

In order to provide a treated-waste product having the required COD, the time between the beginning of the treatment (step a) and the end of the treatment may be at least 160 days, possibly at least 200 days.

In another aspect, the use of the kit of parts according to the present invention for the treatment of alpechin-polluted waste waters produced by oil mills is provided.

In another aspect, a system (10) for the treatment of waste product produced by oil mills is provided. Preferably, said system (10) is suitable for the treatment of alpechin-polluted waste waters. Said system (10) comprises:
- Extracting means (11) for extracting oil from plants or fruits, said extracting means having a daily oil extraction capacity;
- Separating means (12), in fluid communication with said extracting means, for separating oil (21) from waste products (22), the latter being in fluid communication with a retention basin (13) having a capacity of at least 2 fold the capacity in waste products of said extracting means (11);
- A lagoon (14) in fluid communication with said retention basin, said lagoon (14) having a capacity at least 40 fold the capacity of the retention basin (13), preferably at least 50 fold.
Although, strictly speaking, the tem alpechin is restricted to the waste products resulting from pressing olives, said term is used therein to any waste products resulting from any vegetal oil extraction. The present invention is particularly suitable for the treatment of waste products from olive oil extraction.

Preferably, the retention basin (13) may have a capacity of at least 3 fold the capacity in waste products of the extracting means (11). Preferably, the lagoon (14) may have a capacity equivalent to annual capacity in waste products of the extracting means (11).

Said extracting means (11) may be a press. An olive press works by applying pressure to olive paste to separate the liquid from the solid material such as olive mill pomace. The liquid includes the oil and alpechin-polluted waste waters. First the olives are ground into an olive paste using large millstones. After grinding, the olive paste is spread on fiber disks, which are stacked on top of each other, then placed into the press. Traditionally the disks were made of hemp or coconut fiber, but can also be made of synthetic fibers. These disks are then put on a hydraulic piston, forming a pile. Pressure is applied on the disks, thus compacting the solid phase of the olive paste and percolating the liquid phases (oil and alpechin-polluted waste waters). In that case, separating means (12) of the present system may be decanter or vertical centrifuge. Said oil (23) is then separated from alpechin-polluted waste waters.

Said extracting means (11) may also be a decanter allowing the extraction and separation of oil, alpechin-polluted waste waters and solid material such as olive mill pomace by centrifugation. In that case the olives are crushed to a fine paste. This can be done by a hammer crusher, disc crusher, depitting machine or knife crusher. Afterwards the paste is pumped in to an industrial decanter where the phases will be separated. Water is added to facilitate the extraction process with the paste. The decanter may be a horizontal centrifuge. The phases will be readily separated according to their different densities (pomace > alpechin > oil). The separating means to separate oil and alpechin may be a vertical centrifuge.

The separating means (12) are in fluid communication with a retention basin (13). Once oil and alpechin are separated, alpechin is sent to said retention basin (13). As previously mentioned, the capacity of the retention basin (13) depends on the daily capacity of the extracting means.

A real size test was carried out. One day before the beginning of the olive oil extraction, the retention basin (13) may be filled with a solution containing at least one bacterium (1) from *Bacilli* class. The retention basin is also provided with an aeration or oxygenation device which may work continuously or for at least five hours per day. Aeration or oxygenation will also ensure homogenous mixing of the solution within said retention basin (13). The daily amount of alpechin provided by the separating means (12) was then added to the retention basin to form the waste solution. The pH of the alpechin ranged usually from 4.5 to 5.5. Therefore, pH regulator (4) such as sodium hydroxide was added to said waste solution as well as enzymes (2). The pH within the retention basin (13) was maintained between 6 and 9 which was required to favor sufficient enzymatic activity. Injections of the pH regulator (4) and of enzymes (2) were automatically controlled. For example, when 30 tons of olives were pressed, 6 cubic meter of alpechin were obtained and then 600 ml per day of a solution of enzymes (2) were added. The amount is calculated based on the capacity of the extracting means and thus on the amount of alpechin produced. Enzymes (2) were added to the retention basin as soon as alpechin from daily olive press was added. The alpechin (22) was added to the retention basin (13) by the bottom thereof. When, the retention basin (13) was full, the alpechin-surplus overflew and flew down to the lagoon (14) along a channel fluidly communicating the retention basin (13) and said lagoon (14). When the capacity of the retention basin (13) was equivalent to around three fold the daily amount of alpechin, the overflow started after three days and part of the waste solution flew into the lagoon (14). After ca 50 days, a solution comprising the fungus (12 liter per day), supplemented with urea, was added to the retention basin (13). Said fungus solution may be also added to the lagoon (14). Addition of fungus was daily repeated until the end of alpechin inflow. The waste solution was then let to rest in the retention basin and the lagoon to provide a treated-waste product, or treated-alpechin, having a chemical oxygen demand lower than 125 mg/l. The time to achieve the required COD amount of said treated-waste product was around 200 days. The time needed depends *inter alia* on the concentration used and the dimension of the system (retention basin and lagoon) or the annual capacity in waste products in the extracting means (11). Preferably, the treated-waste product may have a chemical oxygen demand lower than 110 mg/l.

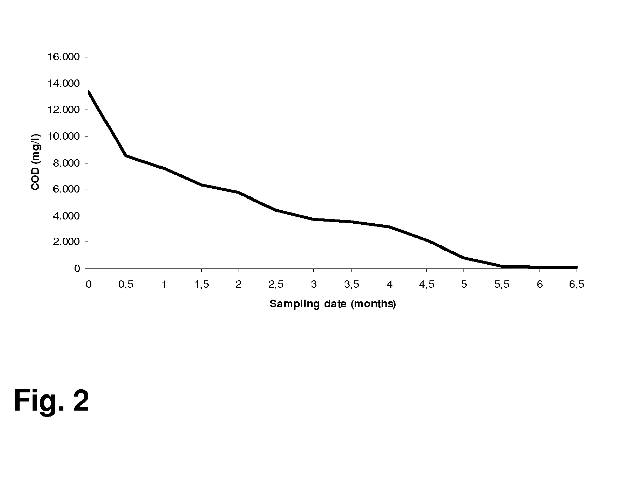

Fig. 2 represents the evolution of COD in function of time when the process according to the present invention is applied in conditions detailed above. The cocktail of bacteria was *Bacillus subtilis* ATB-BS-10, *Bacillus subtilis* ATB-BS-13, *Bacillus lichenoformis* ATB-BS-14, *Bacillus sporothermodurans* ATB-BS-12 and *Bacillus sporothermodurans* ATB-BS-1 5. Enzymes used were alpha-amylase and neutrases from *Bacillus amyloliquefaciens.* The fungus used was *Phanaerochete Chrysosporium.* Every two weeks, a sample from the lagoon was analyzed to determine the chemical oxygen demand. Results are listed in Table 1 below.

**Table 1**

| *Sampling Date (months)* | *COD (mg*/*L)* |
|---|---|
| 0 | 13385 |
| 0.5 | 8520 |
| 1 | 7570 |
| 1.5 | 6340 |
| 2 | 5760 |
| 2.5 | 4440 |
| 3 | 3740 |
| 3.5 | 3510 |
| 4 | 3180 |
| 4.5 | 2120 |
| 5 | 830 |
| 5.5 | 205 |
| 6 | 115 |
| 6.5 | 105 |

After 6.5 months, the chemical demand of oxygen measured in the lagoon was lower than 110mg/l. This strongly highlights that the present invention allows treating and purifying waste product produced by oil mills.

## Claims

1. Composition comprising waste products produced by oil mills, enzymes (2) and at least one polyphenol-resistant bacterium (1), preferably said bacterium comprises a bacterium from *Bacilli* class.

2. Composition according to claim 1 further comprising one or more fungi (3), preferably from *Basidiomycetes* class, and optionally a pH regulator (4).

3. Kit of parts for the treatment of waste product produced by oil mills comprising at least one polyphenol-resistant bacterium (1) and enzymes (2).

4. Kit of parts according to claim 3 further comprising one or more fungi (3), preferably from *Basidiomycetes* class.

5. Kit of parts according to claims 3 or 4 wherein said at least one polyphenol-resistant bacterium (1) is selected from *Bacilli* class, preferably from *Bacillus* genus.

6. Kit of parts according to claims 4 or 5, wherein said one or more fungi (3) are from *Phanerochaete* genus.

7. Kit of parts according to any of previous claims 3 to 6 **characterized in that** it comprises enzymes (2) selected from the group consisting of proteases, amylases, alcalases and neutrases or mixtures thereof.

8. Kit of parts according to any of previous claims 3 to 7 **characterized in that** it comprises a cocktail of at least four bacteria, preferably comprising *Rhodococcus erythropolis, Acinetobacter johnsonii,* and five bacteria from *Bacilli genus.*

9. Kit of parts according to any of previous claims 3 to 8 **characterized in that** it further comprises a pH regulator (4).

10. Kit of parts according to any of previous claims 3 to 9 **characterized in that** it further comprises nutrients for said at least one polyphenol-resistant bacterium (1).

11. Kit of parts according to any of previous claims 3 to 10 **characterized in that** it further comprises one or more gram-positive bacterium or one or more gram-negative bacterium.

12. A process for the treatment of waste products produced by oil mills comprising the steps of:
a) contacting the waste product with at least one polyphenol-resistant bacterium (1) to form a waste solution,
b) adding enzymes (2) and, optionally, a pH regulator (4) to maintain the pH of the solution between 6 and 9,
c) adding one or more fungi (3) from *Basidiomycetes* class, and let rest for a time sufficient to provide a treated-waste product having a chemical oxygen demand lower than 125 mg/l, preferably lower than 110mg/l.

13. A process according to claim 12 **characterized in that** it comprises the preliminary step of preparing a medium suitable for the growth of said at least one bacterium (1).

14. A process according to claim 12 or 13 **characterized in that** said one or more fungi (3) is added from 30 to 70 days after the beginning of the step a), preferably from 40 to 60 days after the beginning of the step a).

15. System (10) for the treatment of waste products produced by oil mills comprising:
- Extracting means (11) for extracting oil from plants or fruits, said extracting means (11) having a daily oil extraction capacity;
- Separating means (12), in fluid communication with said extracting means, for separating oil (21) from waste products (22), the latter being in fluid communication with a retention basin (13) having a capacity of at least 2 fold the capacity in waste products of said extracting means (11),
- A lagoon (14) in fluid communication with said retention basin (13), said lagoon (14) having a capacity at least 40 fold the capacity of the retention basin (13), preferably at least 50 fold.
